# EUROPEAN PATENT APPLICATION

(11) **EP 1 537 873 A1**
(43) Date of publication of application: **08.06.2005**
(21) Application number: 02771999.6
(22) Date of filing: 29.09.2002
(51) Int. Cl.: A61K 35/78, A61K 35/64, A61P 25/30, A61P 25/36

(54) **THE PHARMACEUTICAL FORMULATION OF TRADITIONAL CHINESE MEDICINE FOR ABSTINENCE OF DRUG AND PREPARATION METHOD AND USE THEREOF**

(30) Priority: 21.06.2002 CN 02123741
(71) Applicant: Titan Pharmaceuticals (Shenzhen) Ltd, Guangdong 518001 (CN)
(72) Inventor: FANG, Wenquan, Shenzhen, Guangdong 518001 (CN)
(74) Representative: Graf von Stosch, Andreas, Dr. rer. nat.
(86) International application number: PCT/CN2002/000697
(87) International publication number: WO 2004/000341

(57) **Abstract**

The present invention relates to compound Chinese medicine preparation, and is especially one drug addiction stopping Chinese medicine prepared with red sage, mankshood, cassia, amomum fruit and other Chinese medicinal materials. The present invention also discloses its preparation process and application.

## Description

### BACKGROUND OF THE INVENTION

### 1. Field of the Invention

The present invention relates to compound Chinese traditional medicine preparation, particularly to one Chinese traditional medicine for stopping drug addiction prepared with panax ginseng root, aconitum carmichaeli debx root, cinnamomum cassia stem bark and amomum villosum fruit etc. The present invention also discloses its preparation procedure and application.

### 2. Description of Prior Art

The present invention relates to Chinese traditional medicines for stopping drug addiction. What is called stopping drug addiction is to relief or refrain from a withdrawal symptoms which appears after intermitting use of addictive narcotic drugs such as opium. The opium is a kind of drug such as diamorphine, cocaine, morphia, bhang etc., which has a function of easing pain and anaesthesia and a user may rely on it not only physically but psychologically. Opium is bitter, warm and dry. When smoking opium at initial stages, a drugger may have a sense of pleasure. The inhalational smoke may spread over inter body of the drugger, and he himself may feel very comfortable from head to foot. Meanwhile the drugger is addicted to the opium gradually. His internal organs and spirit figs up, and arms and legs are peaceful after smoking. Smoke with the power of fire is inhaled into lung, spreading veins and arteries, swimming in blood, spreading internal organs, circumfluent the whole body. If a person has a long-term freak-out, poison harms may stay with him, consume and wound his spirit and blood, damage his internal organs. The harms of the opium are known worldwide and have become social effects of pollution, so opium-smoking should be forbidden.

There are two traditional methods for stopping drug addiction. One is to use medicine which can assuage pain strongly, such as methadone and roetorphine, which shall be decreased dose gradually. The drugger has light pains somewhat at incipience, but the treatment period is too long to decrease dose, and new dependence of the drugger may appear. The other method is to use non-dope, such as anilin imidazole clonidine and lofexidine. These medicines are effective for various withdrawal symptoms, and the treatment period is short, and no new addiction will appear. But the method requires large quantity dose and has high incidence rate.

Additionally, there are reports concerning Chinese medicines for stopping drug addiction, such as the Chinese medicine "Dongtang drug addiction stopping capsule" [Chinese medicine magazine-1998, (6)], which is prepared with Polygala telephioides, Cordyceps, root of Co donopsis Pilosula, Chinese angelica root and tuber of elevated gastrodia; "Drug addiction stopping capsule" [Chinese examination prescription -1997, (6)], which is prepared with capejasmine, aucklandia root, rhubarb and corydalis tuber; "Yanyang drug addiction stopping pill" [Guiyan college of traditional Chinese medicine transaction-1995, (1)], which is prepared with Corydalis tuber, datura flower and epimeddium; "Fufang Dongyuan Gao"[Chinese medicine and Western medicine combined magazine-1995, (9)], which is prepared with Corydalis turtschaninovii tuber, Ramulus Uncariae Cum Uncis, astragalus root and Cordyceps; "Fukang capsule" [Chinese medicine drug addition message-1995, (4)], which is prepared with astragalus, corydalis tuber, rhizome of Chinese monkshood, Datura fruit and Cordyceps; "Rescue enthrallment and drug addiction stopping pill" [Gansu science and technology information -1994, (3)], which is prepared with root of american ginseng, corydalis tuber, angelica polymorpha root, bupleurum root and cinnamomum cassia stem bark; "Humen mixture" [Beijing Chinese medicine university transaction-1996, (4)], which is prepared with root of large-flowered skullcap, goldthread root, Ejiao and root bark of barbary wolf berry; "Yijieling" [Nanjing Chinese medicine university transaction-1997, (4)], which is prepared with prepared Sichuan aconite root, dried ginger, panax ginseng root, herb of seoul wildginger and bark of amur corktree. Additionally, prescriptions of Chinese medicine for stopping drug addiction are also published in China patents, such as Patent No.CN00114357, CN00120522, CN0109433, CN00121305, CN97116036, CN97108008, CN99103255 and CN95114006.

Some of the above-mentioned medicines cannot cure effectively, some are prepared with expensive components, and some are intermitted to develop because of ineffective curative effect.

The present invention is to provide a compound Chinese medicine for stopping drug addiction which is of stable efficacy, safety and convenience, minimal side effect and low price.

### SUMMARY OF THE INVENTION

The present invention is to provide a compound Chinese medicine preparation which can refrain from opium withdrawal symptoms. The Chinese medicine of the present invention can be cooperated with traditional substitute and degression therapy in order to relief or refrained from the opium withdrawal symptoms, and to effect abstinence from drugs.

The Chinese medicine of the present invention is prepared with panax ginseng root and aconitum carmichaeli debx root, which is the warm components. The two can nourish spleen and kidney, nourish premordial energy greatly, and consolidate origin, so they are dominant drugs of the prescription. Cinnamomum cassia stem bark, amomum villosum fruit and schizandra chinensis fruit are mixed to assist the panax ginseng root and aconitum carmichaeli debx root to nourish spleen and kidney more effectively, and they three drugs also have efficacies such as relieving darrhea, arresting sweating, arresting seminal emission and tranquilization. Additionally, angelica polymorpha root and paeonia lactiflora root are mixed therein to assist the basic remedy to nourish female blood, invigorate the circulation of blood, activate the channels, nourish the liver to relieve pain and relieve convulsion. Moreover, salvia miltiorrhiza root and zizyphus jujuba seed are mixed therein to assist the basic remedy to relief fidget, enriching the blood, soothe the nerves, arresting sweating and hidroschesis. Further more, glycyrrhiza uralensis root is mixed therein to supplement vital energy, invigorate the middle-warmer, nourish the lung arrests cough, antidote the poison, assuage pain and concoct the property of the medicine, and to be a cionductant drug. The Chinese medicine preparation of the present invention adapts to the drugger who has insufficient spleen and kidney, insufficient vital energy and blood, blood stasis and vital energy retardation and evil poison disordering heart. Such pathogenesis belongs to false and true met and cold and heat interwoven, so according to the principle of Chinese medicine dialectical abstinence from drugs, the treatment principle may be adopted which has the efficacy of warming spleen and kidney, benefiting vital energy, enriching the blood, promoting flow of qi and blood circulation, relieving mental stress and abstinence from drugs.

The effect of the Chinese medicine for stopping drug addiction of the present invention is proved by clinical observations by drug-relief reformatories of Shanxi' Weinan, Xian and Yanta in China. Also, China drug dependence research institute has tested the Chinese medicine preparation of the present invention on that of long drug and rapid drug. The long drug is tested on a rat which is dosed 150g/kg one time, but there is no toxic symptom on the rat and also no death of it. So the biggest dosage for the rat one time is beyond 150g/kg. The biggest dosage for endurance is fixed on 15g/kg and may not be increased for concentration of chemical and injection capacity. The toxic reaction can generate and the embodiment is the hurt on target organs, such as alimentary canal, liver and stomach if a rat and a dog are dosed significant dosage (the rat is dosed with more than 100g/kg, the dog is dosed more than 30g/kg) for a long time. But there is no toxic reaction if a rat is dosed less than 75k/kg and a dog is dosed less than 20k/kg continuously for two months. In addition to the number of the leucocytes of several animals in the group of significant dosage is on the high side by a hematological examination, there is no abnormity on every indicator. Moreover, there is no significant difference on the average of indicators which are tested in all groups by a blood biochemical examination.

Xian medical College has enacted a quality specification for the Chinese medicine preparation for stopping drugs addiction of the present invention. In Accordance with the abstracted technology, five essential components are picked out as a quality index among the chemical compositions of the medicine by quality specifications. Aconitine is one essential component of Aconitum carmichaeli Debx root (concoct), content of which is controlled for administer drugs' safety. Moreover, Panax saponin is one essential component of the principal drug Panax ginseng root that is abstracted for a content measurement standard, and the control index is constituted on the character, power of hydrogen and relative density of the component. Three batch numbers are selected by primary stability test on the Chinese medicine for stopping drug addiction of the present invention, which are file sample observation for three months and examined with the standard, provision such as every physics and chemistry indexes and hygiology examination. It is explained the Chinese medicine for stopping drug addiction of the present invention has stable quality.

The Chinese medicine for stopping drug addiction of the present invention comprises: (quantity of crude drugs in every 100 dose prepared medicine)
Panax ginseng root 68-205g;
Aconitum carmichaeli Debx root (concoct) 75-225g;
Cinnamomum cassia stem bark 38-112g;
Amomum villosum fruit 38-112g;
Schizandra chinensis fruit 31-93g;
Angelica polymorpha root 56-168g;
Paeonia lactiflora root 75-225g;
Cyperus rotundus root 62-187g;
Corydalis turtschaninovii tuber 75-225g;
Buthus martensii Karsch 31-93g;
Salvia miltiorrhiza root 75-225g;
Zizyphus jujuba seed 112-337g;
Glycyrrhiza uralensis root 62-187g.

A preferred prescription comprises: (quantity of crude drugs in every 100 dose prepared medicine)
Panax ginseng root 102-171 g;
Aconitum carmichaeli Debx root (concoct) 112-187g;
Cinnamomum cassia stem bark 56-94g;
Amomum villosum fruit 56- 94g;
Schizandra chinensis fruit 47-78g;
Angelica polymorpha root 84-140g;
Paeonia lactiflora root 112-187g;
Cyperus rotundus root 94-156g;
Corydalis turtschaninovii tuber 112-187g;
Buthus martensii Karsch 47-78g;
Salvia miltiorrhiza root 112-187g;
Zizyphus jujuba seed 169-281g;
Glycyrrhiza uralensis root 94-156g.

A best preferred prescription comprises: (quantity of crude drugs in every 100 dose prepared medicine)
Panax ginseng root 137.5g;
Aconitum carmichaeli Debx root (concoct) 150.0g;
Cinnamomum cassia stem bark 75.0g;
Amomum villosum fruit 75.0g;
Schizandra chinensis fruit 62.5g;
Angelica polymorpha root 112.5g;
Paeonia lactiflora root 150.0g;
Cyperus rotundus root 125.0g;
Corydalis turtschaninovii tuber 150.0g;
Buthus martensii Karsch 62.5g;
Salvia miltiorrhiza root 150.0g;
Zizyphus jujuba seed 225.0g;
Glycyrrhiza uralensis root 125.0g.

The dose type of the Chinese medicine for drug stopping addiction of the present invention may be troche, capsule, oral liquid, decoction, buccal tablets, granula, pill, powder, suspensoid, solvent, drop and drop pill, and the preferred one is oral liquid. Carriers of the drugs may be starch, saccharose, lactose, fructose, other sugar, cellulose derivatives, β-cyclodextrin, phosphatide material and other traditional supplementary materials may be added therein, such as wetting and lubricant. The drug of the present invention can be dosed orally by three times one day, 2-3 doses one time, and the dose may be decreased gradually.

The Chinese medicine for stopping drug addiction of the present invention may be prepared by the following method: cyperus rotundus root, amomum villosum fruit, angelica polymorpha root and cyperus rotundus root are dipped in water for one and a half hours, the quantity of water is eight times as much as the four components, volatile oil (yield is 0.26-0.41%) gained by distilling for one and a half hours and distillate (collection ratio is 1:1) are added with tween 80 (prepared) 80 (0.5% of the preparation quantum) to make up a mixture, then, the mixture is preserved in another container for standby. Coarse poder of panax ginseng root is mixed with ethanol which has 75% concentration to heat and circumfluent for three times, the quantity of the ethanol is five times, four times and five times respectively, the time for heating is four hours, two hours and two hours respectively. Then filter the retrieve ethanol (ratio of getting is about 8-10%) for standby after mixture, cold storage and standing the circumfluence for twenty-four hours. Dregs of buthus martensii karsch is decoct with water which is six times as much as that of the dregs for three times and every time is kept for thirty minutes, after decocted and poached, the filtrate is combined and heated up to concentrate until the relative density reaches 1.10-1.20 (50°C), then the percolate is added and mixed, next is ethanol with the density of 95%, when the density of the ethanol is changed to 70%, cold storage and standing it for twenty-four hours, then filtrate, and reclaim the ethanol in the filtrate until there is no smell of the ethanol for standby. The residual medication of the primary anagraphy is mixed with the abstracted dregs (except for Buthus martensii Karsch) then dipped in water for one and a half hours, the mixture is decocted for three times and the time of every times are three hours, two hours and one and a half hours respectively, the quantity of the added water is eight times, six times and six times respectively. The filtrate is combined and heated up to concentrate until the relative density reaches 1.10-1.20 (50°C), ethanol with the density of 95% is added therein, when the density of the ethanol is changed to 65%, to cold storage and standing it for twenty-four hours, then filtrate, and reclaim the ethanol in the filtrate, and concentrate until the relative density reaches 1.10-1.20 (50°C), water which is three times as much as the filtrate is added to mix round, then filter after standing for twenty-four hours, the filtrate and the abstracted dregs of the mentioned medicinal materials are mixed up with caustic soda dissolving which concentration is 10% to make the PH value be PH6, then add distilled water to 1000ml, the sequential steps are pouring in, covering, sterilization and packing.

A clinical validity research on the Chinese medicine for stopping drug addiction of the present invention is by the following experiment:

### Experimental program:

1. Experimental design: polycentric open trial.
2. Selecting standard: ages from sixteen to sixty, unisex.
   The patient for the drug-addiction stopping treatment must have a middle rate of dependency on opium and must accord with DSM-IV opium dependency treatment standard. He also should have obvious withdrawal symptoms and have freaked-out all the times before the treatment. His urine specimen minim morphine test is positive or naloxone urgency test is positive, but the freaked-out method is on limit.
   When The Chinese medicine for stopping drug addiction is used in prostrated withdrawal symptoms, and prostrated withdrawal symptoms after the treatment is marked more than 12.
3. Excluding standard: a person who is a psychopath, or his heart, liver and renal has serious failure, or body wastes seriously, or a gravida, or a women in lactation period.
4. Dosed for test: the decoction of the present invention (in short: YAD) with dosage of 10ml, hydrochloric acid Methadone decoction (in short: Methadone).
5. Dose project
6. Used for detoxification treatment
   YAD: adopted for a middle rate of dependency on opium. From day 1 to day 3: 2-3 bottle/time, 3 times/day; from day 4 to day 10: 1 bottle/day, 3 times/day.
   Combined treatment group: adopted for a serious rate of dependency on opium. Day 1 to day 3: methadone 30-50 mg/day, YiAn decoction 2-3 bottles/time, 3 times/day. Day 4 to day 10: Yi An 1 bottle/time, 3 times/day.
7. Used for prostrated withdrawal symptoms treatment after the ten-day detoxification. YAD 1 bottle/time.
   Day 11 to day20: 3 times/day.
   Day 21 to day 30: 2 times/day.
   Day 31 to day 40: 1 time/day.
8. Grade record
   The medical care personnel are trained with the clinical trial project and pretest of a unified standard of perfection before the test. The recording is performed by a specially-assigned person, and the recording of every test groups is recorded by both a technician and medical care personnel of local drug-relief reformatory. The content includes:

### Brief case history

Withdrawal symptoms assessment scale: the assessment is performed an hour after dosing every day in the morning before the treatment and in the treatment. clinical symptom is graded into five levels, severity is graded into four levels, that are 0 level (no reaction), I level (light), for which a patient has symptom by enquiry; II level (moderate), for which a patient can pour out the symptom and can tolerate the symptom; III level (serious), for which a patient can not tolerate the symptom. Hamilton anxiety scale (HAMA): to test three times and one time when the drug addiction breakout before the test, one time on the fifth day in the treatment, one time at the end of the treatment. Type matter can be added in remark when a patient appears special psychological block.

Prostrated withdrawal symptoms assessment scale: to test every five days and begin on the eleventh day. The clinical symptom is graded into four levels according to severity, which includes none (no syndrome), light (syndrome can not be noticed or sensed), moderate (syndrome can be noticed easily), serious (syndrome is seriously and can not be tolerated).
9. Assessment index of curative effect

Marks of detoxification (total marks of asking earnestly, anxiety and insomnia is less than ten. Each level of withdrawal symptoms marks is zero);

Total assessment marks of assessing daily for withdrawal symptoms; assessment marks of assessing daily for primary withdrawal symptoms; and anxiety scale assessment and so on. No obvious withdrawal symptoms when therapy is discontinued, intramuscular injection naloxone with 0.4-0.8 mg for pressing addiction test and the result is negative or emiction opium sample examination is negative. If the emiction examination is positive or questionable, naloxone for intramuscular injection should be 0.8mg.

### Result of the treatment

### Analysis for detoxification effect:

Referring to Table 1, in the YAD group, 154 patients were detoxified within 3 - 4 days (31.11%). 98.78% of the patients were detoxified within 10 days. In the combined treatment group, 29 patients were detoxified within 3 - 4 days (38.67). 94.67% of the patients were detoxified within 10 days. In both groups, over 60% of the patients were detoxified within 5 - 7 days.

**Table 1**

| Detoxification Analysis of the YAD Group and Combined Treatment Group | | | | |
|---|---|---|---|---|
| Time required for complete detox (days) | YAD Group | (n=495) | Combined treatment group 1 (n=75) | |
| | n | % of total | n | % of total |
| Day 3-4 | 154 | (31.11) | 29 | (38.67) |
| Day 5-7 | 170 | (65.45) | 19 | (64.00) |
| Day 8 | 100 | (85.65) | 8 | (74.67) |
| Day 9 | 54 | (96.56) | 11 | (89.34) |
| Day 10 | 11 | (98.78) | 4 | (94.67) |
| Withdrawal marks >10 | 6 | (1.21) | 4 | (5.33) |
| Judgments standards: (1) craving + anxiety + sleepless were < 10. (2) The marks of all the other withdrawal symptoms were 0. | | | | |

### Analysis for total marks of detoxification:

Table 2 shows daily changes of total marks of detoxification of the group YAD and, which are in the treatment.

The difference of the total marks of daily detoxification is obvious between none-dosing and dosing in both groups, which has statistics meaning (p<0.001). Since the combined treatment group 2 is through the treatment period of Methadone, the total marks of detoxification decreases to 11.12±5.52 when only YIAN decoction is used. And from on the third to fifth day after using YIAN decoction, total marks of detoxification will decrease greatly, as shown in Table 3.

**Table 2**

| Daily changes of the total marks during the treatment of YAD and Combined group 1 | | |
|---|---|---|
| Items | YAD Group (n=495) (X ± S) | Combined treatment group 1 (n=75) (X ± S) |
| Before the treatment | 99.74 ± 13.38 | 55.48 ± 40.84 |
| Day 1 after the treatment | 84.92 ± 18.20 | 37.35 ± 39.54 |
| Day 2 | 64.66 ± 19.05 | 30.56 ± 28.80 |
| Day 3 | 47.85 ± 18.90 | 24.77 ± 21.19 |
| Day 4 | 34.60 ± 15.98 | 20.27 ± 15.12 |
| Day 5 | 25.59 ± 13.56 | 16.82 ± 9.77 |
| Day 6 | 18.40 ± 11.01 | 14.60 ± 6.52 |
| Day 7 | 13.38 ± 8.78 | 12.56 ± 5.28 |
| Day 8 | 8.96 ± 6.39 | 10.57 ± 4.16 |
| Day 9 | 5.78 ± 4.38 | 8.77 ± 4.41 |
| Day 10 | 4.81 ± 15.20 | 7.08 ± 2.74 |
| P<0.001 when compared before dose and after dose in both groups. | | |

**Table 3**

| Daily Changes of the Total Marks during the Treatment of the Combined Treatment Group 2 | |
|---|---|
| Items | Combined treatment group 2 (n=40) (X ± S) |
| Before the treatment | 11.12 ± 5.32 |
| Day 1 after the treatment | 11.42 ± 4.97 |
| Day 2 | 9.72 ± 3.76 |
| Day 3 | 8.28 ± 3.62 ** |
| Day 4 | 5.18 ± 2.12 *** |
| Day 5 | 4.71 ± 1.58 *** |
| ** P<0.01 *** P<0.001 when compared before dose and after dose | |

### Daily changes of primary withdrawal symptoms:

Table 4 shows the daily decrease of marks of primary withdrawal symptoms in the groups of YAD and combined treatment group 1. The primary withdrawal symptoms are hungriness, anxiety, lachrymation, ostealgia and myalgia, abdominal pain and loose bowels and insomnia, when compared the marks of before dosing with that of after dosing, the difference of the YAD group is obvious (P<0.001), for the combined treatment group 1, except for the instance of abdominal pain and loose bowels has unobvious difference, others have obvious difference (P<0.01 P<0.001).

**Table 4**

| Daily Decreases of the Major Withdrawal Symptoms in the YAD Group | | | | | | |
|---|---|---|---|---|---|---|
| Major withdrawal symptoms (n=495) | | | | | | |
| Days of treatment | Craving | Anxiety | Tearing | Bone and muscle pain | Abdominal pain and diarrhoea | Sleepless |
| Pre-treatment | 2.98 ± 0.10 | 2.78 ± 0.41 | 5.35 ± 1.04 | 7.99 ± 1.61 | 4.38 ± 2.92 | 11.73 ± 1.10 |
| Post day 1 | 2.80 ± 0.49 | 2.56 ± 0.58 | 4.39 ± 1.39 | 7.30 ± 1.81 | 3.70 ± 2.90 | 10.83 ± 2.11 |
| Day 2 | 2.48 ± 0.81 | 2.18 ± 0.79 | 3.02 ± 1.54 | 6.12 ± 1.96 | 2.74 ± 2.52 | 10.15 ± 2.54 |
| Day 3 | 2.18 ± 1.04 | 1.72 ± 0.89 | 1.98 ± 1.65 | 5.08 ± 2.12 | 2.05 ± 2.44 | 9.15 ± 2.82 |
| Day 4 | 1.86 ± 1.14 | 1.22 ± 0.81 | 1.21 ± 1.43 | 4.20 ± 2.17 | 1.47 ± 2.18 | 7.99 ± 3.12 |
| Day 5 | 1.59 ± 1.12 | 0.84 ± 0.74 | 0.68±1.13 | 3.45 ± 2.16 | 1.08 ± 1.94 | 7.08 ± 3.13 |
| Day 6 | 1.36 ± 1.04 | 0.55 ± 0.62 | 0.35 ± 0.81 | 2.81 ± 2.11 | 0.61 ± 1.48 | 6.21 ± 3.06 |
| Day 7 | 1.08 ± 0.90 | 0.29 ± 0.49 | 0.15 ± 0.56 | 2.21 ± 1.98 | 0.38±1.15 | 5.32 ± 3.16 |
| Day 8 | 0.85 ± 0.78 | 0.13 ± 0.33 | 0.04 ± 0.28 | 1.58 ± 1.82 | 0.16 ± 0.73 | 4.16 ± 2.87 |
| Day 9 | 0.64 ± 0.66 | 0.06 ± 0.24 | 0.01 ± 0.13 | 1.16 ± 1.63 | 0.04 ± 0.35 | 3.10±2.58 |
| Day 10 | 0.47 ± 0.64 | 0.03 ± 0.18 | 0.00 ± 0.00 | 0.82 ± 1.44 | 0.01 ± 0.13 | 2.40 ± 2.44 |
| Comparison of the major withdrawalal symptoms between pre- and post treatment by the YAD (P<0.001). | | | | | | |

### Analysis for change of hungriness:

Referring to table 5, which shows the anxiety scales of before dose and after dose are obvious different in both YAD group and combined treatment group 1 (P<0.001).

**Table 5**

| Hamilton Anxiety Change during the Treatment in Both YAN and the Combined Treatment Group 1 | | |
|---|---|---|
| Items | YAD (n=495) (X ± S) | Combined treatment group 1 (n=75) (X ± S) |
| Pre treatment | 37.11 ± 9.16 | 32.76 ± 10.50 |
| Post treatment day 5 | 13.29 ± 6.96 | 17.11 ± 7.78 |
| Post treatment day 10 | 3.08 ± 3.21 | 7.25 ± 3.72 |
| Comparison of the Hamilton anxiety between the pre- and post treatment groups (P<0.001) | | |

### Effect of treatment for protracted withdrawal symptoms

### Analysis for total marks of the YAD used for the treatment of protracted withdrawal symptoms:

Referring to table 6, the total mark of the protracted withdrawal symptoms was 13.62 ± 3.80 prior to the treatment. The protracted withdrawal symptoms were marked every 5 days during the treatment and the total marks were then compared to the total mark of the pre treatment. A significant difference was observed (P<0.0001).

**Table 6**

| Total Marks of the Protracted Withdrawal Symptoms of the Heroin Dependents Treated by YAD | | | |
|---|---|---|---|
| Observation date | Total marks (X ± S) | t | P |
| Pre treatment | 13.62 ± 3.80 | | |
| Day 6 | 9.43 ± 4.79 | 12.13623 | 0.0001 |
| Day 11 | 5.59 ± 4.43 | 19.51021 | 0.0001 |
| Day 16 | 3.85 ± 4.16 | 22.85605 | 0.0001 |
| Day 21 | 2.23 ± 3.13 | 28.65010 | 0.0001 |
| Day 26 | 1.32 ± 2.29 | 33.54947 | 0.0001 |
| Day 31 | 0.50 ± 1.41 | 36.27168 | 0.0001 |

### Conclusion

The examination of the present invention is designed as a polycentric open trial examination and performed by ten drug-relief reformatories in various areas all over the China by the management and treatment mode of force and free will. On one hand, the aim is to review the coherence and repeatability of examination results of the forced drug-relief reformatories. On the other hand, the aim is to search experiences of Chinese medicine for stopping drug addiction in free will persons, and feasibility of the experiences. The results show that, in forced drug-relief reformatories, the YAD can control the withdrawal symptoms of drug-relier effectively, and the total marks of withdrawal symptoms is decreased apparently after dosing, the primary syndromes of hungriness, anxiety, lachrymation, ostealgia and myalgia, abdominal pain and loose bowels and insomnia are relieved obviously. The effect of stopping drug addiction is affirmative. The effect can be more satisfactory if the methadone is used together in initial days and particularly in initial three days. However, the methadone has dependency and can cause difficulty in quitting the drug or new dependency, even though it bears fruit quickly and controls the withdrawal symptoms totally or completely. Contrarily, Chinese medicine has a lasting efficacious and no dependency, although it bears fruit slowly and controls the withdrawal symptoms incompletely. But if combined, they two can have complementary advantages to overcome their shortcomings.

### DETAILED DESCRIPTION OF THE INVENTION

The Chinese medicine for stopping drug addiction of the present invention will be understood more fully by the following detailed descriptions.

### Embodiment 1

### Preparation of the decoction:

Components of the prescription (quantity of crude drugs in every 100 dose prepared medicine):
Panax ginseng root 137.5g;
Aconitum carmichaeli Debx root (concoct) 150.0g;
Cinnamomum cassia stem bark 75.0g;
Amomum villosum fruit 75.0g;
Schizandra chinensis fruit 62.5g;
Angelica polymorpha root 112.5g;
Paeonia lactiflora root 150.0g;
Cyperus rotundus root 125.0g;
Corydalis turtschaninovii tuber (vinegar bake) 150.0g;
Buthus martensii Karsch 62.5g;
Salvia miltiorrhiza root 150.0g;
Zizyphus jujuba seed (parch) 225.0g;
Glycyrrhiza uralensis root 125.0g.

### Preparation:

Weigh and take the above-descried medicines according to the prescription and place them respectively, then dip Cyperus rotundus root, Amomum villosum fruit, Angelica polymorpha root and Cyperus rotundus root in water for one and a half hour, the quantity of the water is eight times as much as the four components. Volatile oil (yield is 0.26-0.41%) gained by distilling for one and a half hours and distillate (collected ratio is 1:1) are added with tween 80 (prepared) 80 (0.5% of the preparation quantum) to make up a mixture, then, the mixture is preserved in another container for standby. Coarse poder of Panax ginseng root is mixed with ethanol which has 75% concentration to heat and circumfluent for three times, the quantity of the ethanol is five times, four times and five times respectively, the time for heating is four hours, two hours and two hours respectively, then filter the retrieve ethanol (ratio of getting is about 8-10%) for standby after mixture, cold storage and standing the circumfluence for twenty-four hours. Dregs of Buthus martensii Karsch is decocted with water which is six times as much as that of the dregs for three times and every time is kept for thirty minutes, after decocted and poached, the filtrate is combined and heated up to concentrate until the relative density reaches 1.10-1.20 (50°C), then the percolate is added and mixed, next is ethanol with the density of 95%, when the density of the ethanol is changed to 70%, to cold storage and standing it for twenty-four hours, then filtrate, and reclaim the ethanol in the filtrate until there is no smell of the ethanol for standby. The residual medication of the primary anagraphy is mixed with the abstracted dregs (except for Buthus martensii Karsch) then dipped in water for one and a half hours, the mixture is decocted for three times and the time of every times are three hours, two hours and one and a half hours respectively, the quantity of the added water is eight times, six times and six times respectively. The filtrate is combined and heated up to concentrate until the relative density reaches 1.10-1.20 (50°C), ethanol with the density of 95% is added therein, when the density of the ethanol is changed to 65%, to cold storage and standing it for twenty-four hours, then filtrate, and reclaim the ethanol in the filtrate, and concentrate until the relative density reaches 1.10-1.20 (50°C), water which is three times as much as the filtrate is added to mix round, then filter after standing for twenty-four hours, the filtrate and the abstracted dregs of the mentioned medicinal materials are mixed up with caustic soda dissolving which concentration is 10% to make the PH value be PH6, then add distilled water to 1000ml, the sequential steps are pouring in, covering, sterilization and packing.

### Embodiment 2 (quantity of crude drugs in every 100 dose prepared medicine)

Panax ginseng root 137.5g;
Aconitum carmichaeli Debx root (concoct) 150.0g;
Cinnamomum cassia stem bark 75.0g;
Amomum villosum fruit 75.0g;
Schizandra chinensis fruit 62.5g;
Angelica polymorpha root 112.5g;
Paeonia lactiflora root 150.0g;
Cyperus rotundus root 125.0g;
Corydalis turtschaninovii tuber (vinegar bake) 150.0g;
Buthus martensii Karsch 62.5g;
Salvia miltiorrhiza root 150.0g;
Zizyphus jujuba seed (parch) 225.0g;
Glycyrrhiza uralensis root 125.0g.

### Preparation:

Weigh and take the descried medicines according to the prescription and place them respectively, then dip Cyperus rotundus root, Amomum villosum fruit, Angelica polymorpha root and Cyperus rotundus root in water for one and a half hour, the quantity of the water is eight times as much as the four components. Volatile oil (yield is 0.26-0.41%) gained by distilling for one and a half hours and distillate (collected ratio is 1:1) are added with tween 80 (prepared) 80 (0.5% of the preparation quantum) to make up a mixture, then, the mixture is preserved in another container for standby. Coarse poder of Panax ginseng root is mixed with ethanol which has 75% concentration to heat and circumfluent for three times, the quantity of the ethanol is five times, four times and five times respectively, the time for heating is four hours, two hours and two hours respectively, then filter the retrieve ethanol (ratio of getting is about 8-10%) for standby after mixture, cold storage and standing the circumfluence for twenty-four hours. Dregs of Buthus martensii Karsch is decocted with water which is six times as much as that of the dregs for three times and every time is kept for thirty minutes, after decocted and poached, the filtrate is combined and heated up to concentrate until the relative density reaches 1.10-1.20 (50°C), then the percolate is added and mixed, next is ethanol with the density of 95%, when the density of the ethanol is changed to 70%, cold storage and standing it for twenty-four hours, then filtrate, and reclaim the ethanol in the filtrate until there is no smell of the ethanol for standby. The residual medication of the primary anagraphyis mixed with the abstracted dregs (except for Buthus martensii Karsch) then dipped in water for one and a half hours, the mixture is decocted for three times and the time of every times are three hours, two hours and one and a half hours respectively, the quantity of the added water is eight times, six times and six times respectively. The filtrate is combined and heated up to concentrate until the relative density reaches 1.10-1.20 (50°C), ethanol with the density of 95% is added therein, when the density of the ethanol is changed to 65%, cold storage and standing it for twenty-four hours, then to filtrate, and reclaim the ethanol in the filtrate, and concentrate until the relative density reaches 1.10-1.20 (50°C), water which is three times as much as the filtrate is added to mix round, then filter after standing for twenty-four hours, the filtrate and the abstracted dregs of the mentioned medicinal materials are mixed up, and then dye the mixture to dry extract, crush the dry extract as granula which may be taken following its infusion, the granula may be packaged into one hundred packages.

### Embodiment 3 (quantity of crude drugs in every 100 dose prepared medicine)

Panax ginseng root 137.5g;
Aconitum carmichaeli Debx root (concoct) 150.0g;
Cinnamomum cassia stem bark 75.0g;
Amomum villosum fruit 75.0g;
Schizandra chinensis fruit 62.5g;
Angelica polymorpha root 112.5g;
Paeonia lactiflora root 150.0g;
Cyperus rotundus root 125.0g;
Corydalis turtschaninovii tuber (vinegar bake) 150.0g;
Buthus martensii Karsch 62.5g;
Salvia miltiorrhiza root 150.0g;
Zizyphus jujuba seed (parch) 225.0g;
Glycyrrhiza uralensis root 125.0g.

### Preparation:

Weigh and take the descried medicines according to the prescription and place them respectively, then dip Cyperus rotundus root, Amomum villosum fruit, Angelica polymorpha root and Cyperus rotundus root in water for one and a half hour, the quantity of the water is eight times as much as the four components, volatile oil (yield is 0.26-0.41%) gained by distilling for one and a half hours and distillate (collected ratio is 1:1) are added with tween 80 (prepared) 80 (0.5% of the preparation quantum) to make up a mixture, then, the mixture is preserved in another container for standby. Coarse poder of Panax ginseng root is mixed with ethanol which has 75% concentration to heat and circumfluent for three times, the quantity of the ethanol is five times, four times and five times respectively, the time for heating is four hours, two hours and two hours respectively, then filter the retrieve ethanol (ratio of getting is about 8-10%) for standby after mixture, cold storage and standing the circumfluence for twenty-four hours. Dregs of Buthus martensii Karsch is decoct with water which is six times as much as that of the dregs for three times and every time is kept for thirty minutes, after decocted and poached, the filtrate is combined and heated up to concentrate until the relative density reaches 1.10-1.20 (50°C), then the percolate is added and mixed, next is ethanol with the density of 95%, when the density of the ethanol is changed to 70%, to cold storage and standing it for twenty-four hours, then to filtrate, and reclaim the ethanol in the filtrate until there is no smell of the ethanol for standby. The residual medication of the primary anagraphyis mixed with the abstracted dregs (except for Buthus martensii Karsch) then dipped in water for one and a half hours, the mixture is decocted for three times and the time of every times are three hours, two hours and one and a half hours respectively, the quantity of the added water is eight times, six times and six times respectively. The filtrate is combined and heated up to concentrate until the relative density reaches 1.10-1.20 (50°C), ethanol with the density of 95% is added therein, when the density of the ethanol is changed to 65%, cold storage and standing it for twenty-four hours, then filtrate, and reclaim the ethanol in the filtrate, and concentrate until the relative density reaches 1.10-1.20 (50 °C), water which is three times as much as the filtrate is added to mix round, then filter after standing for twenty-four hours, the filtrate and the abstracted dregs of the mentioned medicinal materials are mixed up, and then to dying the mixture to dry extract, crush the dry extract as granula, add amylum, powdered sugar and talcum powder to mix up, encapsulate the NO 1 capsulate, one hundred capsulate can be get.

## Claims

1. A Chinese medicine preparation for curing an withdrawal symptom of addictive drug reliers, every 100 dose of the medicine comprises:
Panax ginseng root 68-205g;
Aconitum carmichaeli Debx root 75-225g;
Cinnamomum cassia stem bark 38-112g;
Amomum villosum fruit 38-112g;
Schizandra chinensis fruit 31-93g;
Angelica polymorpha root 56-168g;
Paeonia lactiflora root 75-225g;
Cyperus rotundus root 62-187g;
Corydalis turtschaninovii tuber 75-225g;
Buthus martensii Karsch 31-93g;
Salvia miltiorrhiza root 75-225g;
Zizyphus jujuba seed 112-337g;
Glycyrrhiza uralensis root 62-187g.

2. The Chinese medicine preparation as claimed in claim 1, wherein every 100 dose of the medicine comprises:
Panax ginseng root 102-171 g;
Aconitum carmichaeli Debx root 112-187g;
Cinnamomum cassia stem bark 56-94g ;
Amomum villosum fruit 56- 94g;
Schizandra chinensis fruit 47-78g;
Angelica polymorpha root 84-140g;
Paeonia lactiflora root 112-187g;
Cyperus rotundus root 94-156g;
Corydalis turtschaninovii tuber 112-187g;
Buthus martensii Karsch 47-78g;
Salvia miltiorrhiza root 112-187g;
Zizyphus jujuba seed 169-281 g;
Glycyrrhiza uralensis root 94-156g.

3. The Chinese medicine preparation as claimed in claim 2, wherein every 100 dose of the medicine comprises:
Panax ginseng root 137.5g;
Aconitum carmichaeli Debx root 150.0g;
Cinnamomum cassia stem bark 75.0g;
Amomum villosum fruit 75.0g;
Schizandra chinensis fruit 62.5g;
Angelica polymorpha root 112.5g;
Paeonia lactiflora root 150.0g;
Cyperus rotundus root 125.0g;
Corydalis turtschaninovii tuber 150.0g;
Buthus martensii Karsch 62.5g;
Salvia miltiorrhiza root 150.0g;
Zizyphus jujuba seed 225.0g;
Glycyrrhiza uralensis root 125.0g.

4. The Chinese medicine preparation as claimed in any one of claim 1 to 3, wherein the medicine adapts to various dose types.

5. The Chinese medicine preparation as claimed in claim 4, wherein the dose types may be troche, capsule, decoction, buccal tablets, granula, pill, powder, suspensoid, solvent, drop and drop pill.

6. The Chinese medicine preparation as claimed in claim 4, wherein the medicine is decoction.

7. The Chinese medicine preparation as claimed in claim 1, wherein the Chinese medicine preparation may be applied in medicine for treating withdrawal symptoms of an addictive drug relier.

8. The Chinese medicine preparation as claimed in claim 1, wherein volatile oil and distillation of cinnamomum cassia stem bark, amomum villosum fruit, angelica polymorpha root and cyperus rotundus root are mixed with abstraction of water or alcohol of Panax ginseng root, Buthus martensii Karsch, Aconitum carmichaeli Debx root, Schizandra chinensis fruit, Paeonia lactiflora root, Corydalis turtschaninovii tuber, Salvia miltiorrhiza root, Zizyphus jujuba seed and Glycyrrhiza uralensis root, drug carrier may be added when necessary.

9. The Chinese medicine preparation as claimed in claim 8, wherein tween 80 is added into the volatile oil and distillation of cinnamomum cassia stem bark, amomum villosum fruit, angelica polymorpha root and cyperus rotundus root; the abstraction of Panax ginseng root, Buthus martensii Karsch, Aconitum carmichaeli Debx root, Schizandra chinensis fruit, Paeonia lactiflora root, Corydalis turtschaninovii tuber, Salvia miltiorrhiza root, Zizyphus jujuba seed and Glycyrrhiza uralensis root is alcohol abstraction.

10. The Chinese medicine preparation as claimed in claim 8, wherein the mixture is adjusted to be of PH6 value using caustic soda, then added water therein.
